# EUROPEAN PATENT APPLICATION

(11) **EP 3 498 298 A1**
(43) Date of publication of application: **19.06.2019**
(21) Application number: 17207602.8
(22) Date of filing: 15.12.2017
(51) Int. Cl.: A61K 45/06, A61K 31/4709, A61K 31/506, A61K 31/522, A61P 9/10, A61P 9/08

(54) **THE USE OF SGC STIMULATORS AND SGC ACTIVATORS ALONE OR IN COMBINATION WITH PDE5 INHIBITORS FOR THE TREATMENT OF BONE DISORDERS INCLUDING OSTEOGENESIS IMPERFECTA (OI)**

(71) Applicant: Bayer AG, 51373 Leverkusen (DE); Bayer Pharma Aktiengesellschaft, 13353 Berlin (DE)
(72) Inventor: GEIß, Volker, 40883 Ratingen (DE); SANDNER, Peter, 42113 Wuppertal (DE); FREY, Reiner, 42113 Wuppertal (DE); STASCH, Johannes-Peter, 81679 München (DE); FOLLMANN, Markus, 50859 Köln (DE)
(74) Representative: BIP Patents

(57) **Abstract**

The present invention relates to sGC stimulators and sGC activators alone or in combination with PDE5 inhibitors or other combination partners for use in the treatment and/or prophylaxis of bone disorders, including osteogenesis imperfecta (OI).

## Description

The present invention relates to sGC stimulators and sGC activators alone or in combination with PDE5 inhibitors or other combination partners for use in the treatment and/or prophylaxis of bone disorders, including osteogenesis imperfecta (OI).

### Background of the invention

Osteogenesis imperfecta (OI) is a group of genetic disorders characterized primarily by extreme bone fragility, fractures and extraskeletal manifestations. OI affects 6 to 7 per 100,000 people worldwide. There are at least eight recognized forms of OI, termed type I, II, III, IV, V, VI, VII and VIII. Types I and IV are the most common forms, affecting 4 to 5 per 100,000 people. The different forms of OI vary considerably in morbidity and diseases severity with Type I be the less and type II the most severe form of OI. The forms are differentiated due to their mutations in the COL1A1, COL1A2, CRTAP, and P3H1 genes. The mutations in the COL1A1 and COL1A2 genes are responsible for more than 90 percent of all cases (van Dijk et al. 2011, Bonafé et al. 2013). Treatment is focusing on symptoms since no disease modifying therapy is available. Besides physical and occupational therapy and prevention measures for fractures and fracture repair also supplementation with vitamin D and calcium is used. In addition, bisphosphonates like pamidronate and zoledronate have become the standard for treatment of children with moderate to severe OI. Bisphosphonates aim to reduce osteoclast activity and lead to an increase in bone mass and to a reduction in fractures and pain (Monti et al. 2014). However, although bisphosphonates are commonly administered in children during long periods of 2-5 years, the optimal treatment regimen and the long-term consequences of this treatment are currently unknown. Further, bisphosphonates do not grow new bone, nor improve the quality of existing bone. Therefore, there is currently no cure for OI and there exists a substantial unmet medical need to improve and prolong life of OI patients.

Further bone disorders include but are not limited to bone fractures and impaired bone healing, with and without surgical procedure. In addition, further bone disorders include rickets, osteomalacia, avascular bone necrosis and Paget disease, with impaired bone remodeling and increased bone fragility and breakdown. Finally bone disorder include different forms of osteoporosis, including but not limited to primary osteoporosis, or secondary osteoporosis, in particular osteoporosis, primary osteoporosis, or secondary osteoporosis which is not caused by a deficiency of sex hormones.

The cyclic nucleotides, cyclic adenosine monophosphate (cAMP) and cyclic guanosine monophosphate (cGMP), were discovered decades ago and represent one of the most important second messenger pathways within cells. It is well established that the regulation of intra-cellular cGMP pools has a substantial impact on physiology and pathophysiology and is one basic principle of pharmacological intervention (Evgenov et al. 2006; Schmidt et al. 2009). Nitrates and PDE5 inhibitors (PDE5i) which could increase intra-cellular cGMP levels are already approved therapies for angina pectoris, pulmonary hypertension (PAH) or erectile dysfunction (ED), respectively. More recently discovered sGC stimulators and sGC activators can overcome significant limitations of nitrates and PDE5i by direct stimulation of the soluble guanylate cyclase. The sGC stimulator riociguat is approved for the treatment of pulmonary hypertension (PAH) and chronic thromboembolic pulmonary hypertension (CTEPH). The sGC stimulator vericiguat is in phase III clinical trials for the treatment of chronic heart failure.

It is well accepted, that sGC stimulators act via direct stimulation of the sGC which does not require NO but requires the prosthetic heme group. Therefore, this compound class of sGC stimulators is defined as NO-independent but heme-dependent sGC stimulators. sGC stimulators bind to the alpha subunit of the non-oxidized and heme-containing sGC (alpha1/beta1), also termed wild type sGC which leads to NO-independent formation and increase of intracellular cGMP (Stasch et al. 2001; Stasch & Hobbs 2009). In addition sGC stimulators enhance the NO-effect on cGMP when NO is bound to the sGC. Therefore, sGC stimulators also exhibit synergistic effects with NO on cGMP production. The indazole derivative YC-1 was the first NO-independent but heme-dependent sGC stimulator described (Evgenov et al. 2006). Based on YC-1, further substances were discovered which are more potent than YC-1 and show no relevant inhibition of phosphodiesterases (PDE). This led to the identification of the pyrazolopyridine derivatives BAY 41-2272, BAY 41-8543 and BAY 63-2521 (Riociguat) (Evgenov et al. 2006). More recently other compound classes were discovered which show different pharmacokinetics but also different organ distribution which might impact on their treatment potential (Follmann et al. 2017). The exact binding site of the sGC stimulators at the wild type sGC is still being debated. If the heme group is removed from the sGC, the enzyme still has a detectable catalytic basal activity, i.e. cGMP is still being formed. The remaining catalytic basal activity of the heme-free enzyme cannot be stimulated by any of the stimulators mentioned above and can also not be stimulated by NO (Evgenov et al. 2006).

This observation is important since heme-free and oxidized forms of the sGC (alpha1/beta1), also termed apo sGC, are preferentially present at diseases which are linked to oxidative stress. The current understanding is that under oxidative stress conditions, the Fe2+ iron atom of the heme group in the beta1 subunit is oxidized to Fe3+ which destabilizes the binding of the heme group to the beta1 subunit and renders the enzyme heme-free. With the discovery of BAY 58-2667 (cinaciguat), a new chemical matter has found which is able to activate heme-free apo sGC. This class is defined as NO-independent and heme-independent sGC activator. Common characteristics of these substances are that in combination with NO they only have an additive effect on enzyme activation, and that the activation of the oxidized or heme-free enzyme is markedly higher than that of the heme-containing enzyme (Evgenov et al. 2006; Stasch JP et al. 2002; Stasch JP et al. 2006). Spectroscopic studies show that cinaciguat displaces the oxidized heme group in the beta1 subunit which, as a result of the weakening of the iron-histidine bond, is attached only weakly to the sGC. It has also been shown that the characteristic sGC heme binding motif Tyr-x-Ser-x-Arg is absolutely essential both for the interaction of the negatively charged propionic acids of the heme group and for the action of cinaciguat. Therefore, it is assumed that the binding site of cinaciguat at the sGC is identical to the binding site of the heme group in the beta1 subunit. (Stasch JP et al. 2006). More recently other classes of sGC activators have been discovered which are different in pharmacokinetics but also in organ distribution which might have an impact on their treatment potential.

It is well established that cGMP increase by sGC stimulators and sGC activators leads to relaxation of vascular smooth muscle cells and a decrease of blood pressure. However, other modes of action beyond vasodilation and targeting the vascular smooth muscle cells are only partly understood and are currently under investigation. In recent years it became obvious that cGMP increase might also have an impact on bone morphology and could potentially stimulate bone growth. It was shown in healthy rodents that treatment with sGC stimulators could lead to bone growth, especially in juvenile rats but this was not observed in adult rats (FDA 2013, summary basis of approval, riociguat, pharmacology review, https://www.accessdata.fda.gov/drugsatfda docs/nda/2013/204819Orig1s000PharmR.pdf, pages 1-3 and 9-20 of the report). However, these investigations were done in healthy animals and do not allow to draw conclusions for the treatment potential of sGC stimulators and sGC activators in bone diseases. WO2002/36120 pertains to increased bone formation in healthy rats, induced by sGC stimulators and claims the use of sGC stimulators for treating osteoporosis. More recently, it has been shown that the sGC activator cinaciguat could increase bone density in ovariectomized female mice, a model for osteoporosis (Joshua et al., 2014). However, cinaciguat differs from later developed sGC activators e.g. in that cinaciguat is a dicarboxylic acid which, compared to more recently developed sGC activators, is expected to show different bone-exposure levels. Further, Joshua et al. applied cinaciguat intraperitoneally in a 10µg/kg bolus dose, which is known to show pronounced effects on blood pressure. Joshua et al. did not show any effect of cinaciguat on the bone structure of non-estrogen-deficient animals. Very recently, the effects on fracture healing of two up-regulators of inducible nitric oxide synthase (iNOS) in a rat model of an open femoral osteotomy: tadalafil, a phosphodiesterase inhibitor, and the nutraceutical COMB-4 (consisting of L-citrulline, Paullinia cupana, ginger and muira puama) have been investigated (Rajfer et al., 2017). The COMB-4 group exhibited a higher maximum strength and higher stiffness, but no significant changes were observed in the tadalafil group and also no significant difference was observed between all groups with respect to callus volume, mineral content and bone density. In contrast, Histing et al. observed that sildenafil accelerates fracture healing in mice (Histing T, et al., 2011).

Accordingly, it is an object of the present invention to provide suitable compounds and compound combinations for use in the treatment and/or prophylaxis of bone diseases, including bone fractures. It is a further object of the present invention to provide suitable compounds for use in the treatment and/or prophylaxis of osteogenesis imperfecta.

It was found that sGC stimulators and sGC activators could be used for the treatment of bone diseases, e.g. for the treatment of osteogenesis imperfecta (OI), bone fractures, impaired bone healing, rickets, osteomalacia, avascular bone necrosis, Paget disease, osteodystrophy, osteopenia, osteolytic lesions produced by bone metastasis, radiotherapy or chemotherapy, periodontitis, hypercalcemia, osteonecrosis, osteosarcoma, osteolytic metastases, familial expansile osteolysis, expansile skeletal and idiopathic hyper phosphatasia, hyperostosis corticalis deformans juvenilis, Camurati Engelmann disease, prosthetic loosening, periprostetic osteolysis, cleiodocranial dysplasia (CCD), multiple myeloma, alveolar bone loss, bone loss caused by immobilization or sex hormone deficiency, bone loss associated with a disease selected from the group consisting of cachexia, anorexia, alopecia, and inflammatory diseases selected from the group consisting of rheumatoid arthritis, psoriatic arthritis, psoriasis, spondyloarthritis, SLE, systemic sclerosis, metastatic cancer and inflammatory bowel disease, osteoarthritis, impaired bone healing after osteotomy, childhood idiopathic bone loss, curvature of the spine, osteoporosis, primary osteoporosis, secondary osteoporosis and in particular osteoporosis, primary osteoporosis, or secondary osteoporosis that is not caused by a deficiency of sex hormones.

One embodiment of the invention is at least one sGC stimulator or sGC activator for use in the treatment and/or prophylaxis of bone diseases selected from the group consisting of osteogenesis imperfecta (OI), bone fractures, impaired bone healing, rickets, osteomalacia, avascular bone necrosis, and Paget disease.

A further embodiment of the invention is at least one sGC stimulator or sGC activator for use in the treatment and/or prophylaxis of osteogenesis imperfecta (OI).

A further embodiment of the invention is at least one sGC stimulator or sGC activator for use in the treatment and/or prophylaxis of bone diseases selected from the group consisting of bone fractures and impaired bone healing.

A further embodiment of the invention is at least one sGC stimulator or sGC activator for use in the treatment and/or prophylaxis of bone diseases selected from the group consisting of rickets, osteomalacia, avascular bone necrosis, and Paget disease.

A further embodiment of the invention is at least one sGC stimulator or sGC activator for use in the treatment and/or prophylaxis of bone diseases selected from the group consisting of osteoporosis, primary osteoporosis, and secondary osteoporosis.

A further embodiment of the invention is at least one sGC stimulator or sGC activator for use in the treatment and/or prophylaxis of bone diseases selected from the group consisting of osteoporosis, primary osteoporosis, and secondary osteoporosis, wherein the osteoporosis, primary osteoporosis, and secondary osteoporosis is not caused by a deficiency of sex hormones.

Compared to these aforementioned findings, inherited bone diseases such as in osteogenesis imperfecta (OI) have different etiologies and it was surprising that sGC stimulators and sGC activators have treatment potential in inherited bone diseases.

In one aspect, the invention discloses compounds and methods for the treatment of bone disease. The term "bone disease" refers to a medical condition that affects the bone. In particular, the bone disease is characterized for example by an aberrant osteoclast activity, an aberrant balance between bone resorption by osteoclasts and bone formation by osteoblasts, or an imbalance in bone metabolism resulting e.g. in bone loss, insufficient bone synthesis bone malformation, or bone instability due to loss of bone density or mass. "Bone loss disorders" or "bone loss associated disorders" include conditions and diseases that are characterized by a net decrease in bone mass and/or bone malformations, and wherein the inhibition of bone loss and rebuilding of bone mass and strengthening of bone structure, such as an improvement of trabecular bone properties and mechanical stability of the bone is desirable.

Therapy and/or prophylaxis of bone diseases may be mediated by bone formation, increase in bone mass (bone anabolic agent), bone healing, improvement of bone structure (trabeculae) and improvement of bone stability.

Within the meaning of the present invention, secondary osteoporosis may be caused by medical conditions including serious kidney failure, cushing's disease, liver impairment, anorexia nervosa and bulimia, (juvenile) rheumatoid arthritis, malabsorption syndromes such as celiac disease, multiple sclerosis, chronic obstructive pulmonary disease (COPD), scurvy, and osteogenesis imperfecta.

Within the meaning of the present invention, secondary osteoporosis may also have hormonal causes including hyperparathyroidism, hyperthyroidism, diabetes mellitus, or hypercortisolism.

Within the meaning of the present invention, secondary osteoporosis may also be caused by thalassemia, multiple myeloma, leukemia, metastatic bone diseases, prolonged inactivity or immobility, or inadequate nutrition (especially lack of calcium and vitamin D).

Within the meaning of the present invention, secondary osteoporosis may also be caused by the following medications or chemicals: cigarette smoking, corticosteroid therapy, anticonvulsants, immunosuppressive agents, alcohol abuse, lithium, aluminum, barbiturates, or antacids containing aluminum.

According to a further embodiment of the present invention, bone diseases are selected from the group consisting of osteogenesis imperfecta (OI), bone fractures, impaired bone healing, rickets, osteomalacia, avascular bone necrosis, Paget disease, and osteoporosis, primary osteoporosis, or secondary osteoporosis, in particular osteoporosis, primary osteoporosis, or secondary osteoporosis which is not caused by a deficiency of sex hormones.

Within the meaning of the present invention, a deficiency of sex hormones is defined as a level of estradiol in men and/or women below 10 pg/mL, or as a level of estradiol below 5 pg/mL (Medscape, https://emedicine.medscape.com/article/2089003-overview; Riggs, L.B. et al., 2002).

It was found that sGC stimulators and sGC activators could be used for the prophylaxis of bone fractures, osteomalacia, osteolytic lesions produced by bone metastasis, radiotherapy or chemotherapy, bone loss due to immobilization or sex hormone deficiency, bone loss associated with a disease selected from the group consisting of cachexia, anorexia, alopecia, and inflammatory diseases selected from the group consisting of rheumatoid arthritis, psoriatic arthritis, psoriasis, spondyloarthritis, SLE, systemic sclerosis, metastatic cancer and inflammatory bowel disease and secondary osteoporosis.

Within the meaning of the present invention, the terms "prevention", "prophylaxis" and "preclusion" are used synonymously in the context of the present invention and refer to the avoidance or reduction of the risk of contracting, experiencing, suffering from or having a disease, a condition, a disorder, an injury or a health problem, or a development or advancement of such states and/or the symptoms of such states.

The treatment or prevention of a disease, a condition, a disorder, an injury or a health problem may be partial or complete.

It was further found that sGC stimulators and sGC activators in combination with PDE5i like sildenafil, vardenafil, tadalafil and/or in combination with compounds employed as standard of care for different bone diseases could be used for the treatment of bone diseases, e.g. for the treatment of osteogenesis imperfecta (OI), bone fractures, impaired bone healing, rickets, osteomalacia, avascular bone necrosis, Paget disease, and osteoporosis, primary osteoporosis, or secondary osteoporosis, in particular osteoporosis, primary osteoporosis, or secondary osteoporosis which is not caused by a deficiency of sex hormones.

Within the meaning of the present invention, current or future standard of care for OI is defined as treatment with compounds selected from the group consisting of compounds which inhibit the breakdown of cyclic guanosine monophosphate (cGMP), such as, for example, inhibitors of phosphodiesterases (PDE) 1, 2 and/or 5, in particular PDE 5 inhibitors such as sildenafil, vardenafil and tadalafil; calcium; vitamin D and metabolites of vitamin D, for example calcitriol; Bisphosphonates, for example Etidronate, Clodronate, Tiludronate, Teriparatide, Pamidronate, Neridronate, Olpadronate, Alendronate, Ibandronate, Risedronate, Zoledronate; strontium ranelate; active ingredients suitable for hormone replacement therapy in osteoporosis, for example estrogen or a combination of estrogen and progesterone; selective estrogen receptor modulators (SERMs), for example raloxifene; parathyroid hormone or analogs of parathyroid hormone, for example teriparatide; modulators of receptor activator of nuclear factor kappa-B ligand (RANKL), for example denosumab; Sclerostin inhibitors, for example romosozumab or BPS804; and TGF-β inhibitors, for example fresolimumab.

It was further found that sGC stimulators and sGC activators in combination with PDE5i like sildenafil, vardenafil, tadalafil or in combination with compounds employed as standard of care for different bone diseases could be used for the prophylaxis of bone diseases, e.g. for the prophylaxis of osteogenesis imperfecta (OI), bone fractures, impaired bone healing, rickets, osteomalacia, avascular bone necrosis, Paget disease, and osteoporosis, primary osteoporosis, or secondary osteoporosis, in particular osteoporosis, primary osteoporosis, or secondary osteoporosis which is not caused by a deficiency of sex hormones.

A further embodiment of the invention is a combination of at least one sGC stimulator or sGC activator and at least one compound selected from the group consisting of inhibitors of phosphodiesterases (PDE) 1, 2 and/or 5, calcium, vitamin D and metabolites of vitamin D, bisphosphonates, selected from etidronate, clodronate, tiludronate, teriparatide, pamidronate, neridronate, olpadronate, alendronate, ibandronate, risedronate, and zoledronate, strontium ranelate, active ingredients suitable for hormone replacement therapy in osteoporosis, selected from estrogen and a combination of estrogen and progesterone, selective estrogen receptor modulators (SERMs), parathyroid hormone and analogs of parathyroid hormone, modulators of receptor activator of nuclear factor kappa-B ligand (RANKL), sclerostin inhibitors, and TGF-β inhibitors for use in the treatment and/or prophylaxis of bone diseases selected from the group consisting of osteogenesis imperfecta (OI), bone fractures, impaired bone healing, rickets, osteomalacia, avascular bone necrosis, and Paget disease.

A further embodiment of the invention is a combination of at least one sGC stimulator or sGC activator and at least one compound selected from the group consisting of inhibitors of phosphodiesterases (PDE) 1, 2 and/or 5, calcium, vitamin D and metabolites of vitamin D, bisphosphonates, selected from etidronate, clodronate, tiludronate, teriparatide, pamidronate, neridronate, olpadronate, alendronate, ibandronate, risedronate, and zoledronate, strontium ranelate, active ingredients suitable for hormone replacement therapy in osteoporosis, selected from estrogen and a combination of estrogen and progesterone, selective estrogen receptor modulators (SERMs), parathyroid hormone and analogs of parathyroid hormone, modulators of receptor activator of nuclear factor kappa-B ligand (RANKL), sclerostin inhibitors, and TGF-β inhibitors for use in the treatment and/or prophylaxis of bone diseases selected from the group consisting of osteoporosis, primary osteoporosis, and secondary osteoporosis, wherein the osteoporosis, primary osteoporosis, and secondary osteoporosis is not caused by a deficiency of sex hormones.

A further embodiment of the invention is a combination of at least one sGC stimulator or sGC activator and at least one inhibitor of phosphodiesterase (PDE) 5, selected from the group consisting of sildenafil, vardenafil, tadalafil and avanafil for use in the treatment and/or prophylaxis of bone diseases selected from the group consisting of osteogenesis imperfecta (OI), bone fractures, impaired bone healing, rickets, osteomalacia, avascular bone necrosis, and Paget disease.

A further embodiment of the invention is a combination of at least one sGC stimulator or sGC activator and at least one inhibitor of phosphodiesterase (PDE) 5, selected from the group consisting of sildenafil, vardenafil, tadalafil and avanafil for use in the treatment and/or prophylaxis of bone diseases selected from the group consisting of osteoporosis, primary osteoporosis, and secondary osteoporosis, wherein the osteoporosis, primary osteoporosis, and secondary osteoporosis is not caused by a deficiency of sex hormones.

According to a further embodiment, the invention provides sGC stimulators for use in the treatment and/or prophylaxis of bone diseases, wherein the sGC stimulator is selected from the group consisting of
- 2-[1-(2-fluorobenzyl)-1H-pyrazolo[3,4-b]pyridin-3-yl]-5-(4-morpholinyl)-4,6-pyrimidinediamine
- 2-[1-(2-fluorobenzyl)-1H-pyrazolo[3,4-b]pyridin-3-yl]-5-(4-pyridinyl)-4-pyrimidineamine
- methyl 4,6-diamino-2-[1-(2-fluorobenzyl)-1H-pyrazolo[3,4-b]pyridin-3-yl]-5-pyrimidinyl(methyl)carbamate (Riociguat)
- methyl 4,6-diamino-2-[1-(2-fluorobenzyl)-1H-pyrazolo[3,4-b]pyridin-3-yl]-5-pyrimidinylcarbamate (Nelociguat)
- methyl {4,6-diamino-2-[5-fluoro-1-(2-fluorobenzyl)-1H-pyrazolo[3,4-b]pyridine-3-yl]pyrimidin-5-yl}carbamate (Vericiguat)
- methyl {4,6-diamino-2-[5-fluoro-1-(2-fluorobenzyl)-1H-pyrazolo[3,4-b]pyridine-3-yl]pyrimidin-5-yl}methylcarbamate
- methyl {4,6-diamino-2-[5-fluoro-1-(2-fluorobenzyl)-1H-pyrazolo[3,4-b]pyridin-3-yl]pyrimidin-5-yl}(2,2,2-trifluoroethyl)carbamate
- 4-amino-2-[5-chloro-3(3,3,3-trifluoropropyl)-1H-indazol-1-yl]-5,5-dimethyl-5,7-dihydro-6H-pyrrolo[2,3-d]pyrimidin-6-one
- 4-amino-2[5-chloro-3-(2,3,6-trifluorobenzyl)-1H-indazol-1-yl]-5,5-dimethyl-5,7-dihydro-6H-pyrrolo[2,3-d]pyrimidin-6-one
- 4-amino-5,5-dimethyl-2-[3-(2,3,6-trifluorobenzyl)1H-thieno[3,4-c]pyrazol-1-yl]-5,7-dihydro-6H-pyrrolo[2,3-d]pyrimidin-6-one
- 4-amino-5,5-dimethyl-2-[3-(2,3,6-trifluorobenzyl)-1H-thieno[2,3-d]pyrazol-1-yl]-5,5-dimethyl-5,7-dihydro-6H-pyrrolo[2,3-d]pyrimidin-6-one
- 4-amino-5,5-dimethyl-2-[7-(2,3,6-trifluorobenzyl)imidazo[1,5-b]pyridazin-5-yl]-5,7-dihydro-6H-pyrrolo[2,3-d]pyrimidin-6-one
- 4-ammo-2-[6-chloro-3-(2,3,6-trifluorobenzyl)imidazo[1,5-a]pyridin-1-yl]]-5,5-dimethyl-5,7-dihydro-6H-pyrrolo[2,3-d]pyrimidin-6-one
- 4-ammo-2-[6-fluoro-3-(2,3,6-trifluorobenzyl)imidazo[1,5-a]pyridin-1-yl]]-5,5-dimethyl-5,7-dihydro-6H-pyrrolo[2,3-d]pyrimidin-6-one
- 4-amino-2-[6-fluoro-3-(2,3,6-trifluorobenzyl)6-fluoroimidazo[1,5-a]pyridin-1-yl]-5,5-dimethyl-5,7-dihydro-6H-pyrrolo[2,3-d]pyrimidin-6-one
- 4-amino-5,5-dimethyl-2-[3-(2,4,6-trifluorobenzyl)imidazo[1,5-a]pyridin-1-yl]]-5,7-dihydro-6H-pyrrolo[2,3-d]pyrimidin-6-one
- 4-amino-2-[3-(2-cyclopentylethyl)imidazo[1,5-a]pyridin-1-yl]-5,5-dimethyl-5,7-dihydro-6H-pyrrolo[2,3-d]pyrimidin-6-one
- 3-(4-amino-5-cyclopropylpyrimidin-2-yl)-1-(2-fluorobenzyl)-1*H*-pyrazolo[3,4-*b*]pyridine (BAY 41-2272)
- 2-{5-fluoro-1-[(3-fluoropyridin-2-yl)methyl]-1H-pyrazolo[3,4-b]pyridin-3-yl}-5-methyl-5-(trifluoromethyl)-4-[(3,3,3-trifluoropropyl)amino]-5,7-dihydro-6H-pyrrolo[2,3-d]pyrimidin-6-one
- *ent*-*N-*[(2S)-amino-2-methylbutyl]-8-[(2,6-difluorobenzyl)oxy]-2,6-dimethylimidazo[1,2-a]pyridine-3-carboxamide (enantiomer A)
- *ent*-*N*-(2-amino-2-methylbutyl)-8-[(2,6-difluorobenzyl)oxy]-2,6-dimethylimidazo[1,2-a]pyridine-3-carboxamide (enantiomer B)
- *ent*-N-(2-amino-5,5,5-trifluoro-2-methylpentyl)-2,6-dimethyl-8-[(2,3,6-trifluorobenzyl)oxy]imidazo[1,2-a]pyridine-3-carboxamide (enantiomer B)
- *ent*-N-(2-amino-5,5,5-trifluoro-2-methylpentyl)-8-[(2,6-difluorobenzyl)oxy]-2,6-dimethylimidazo[1,2-a]pyridine-3-carboxamide (enantiomer B)
- *ent*-N-(2-amino-5,5,5-trifluoro-2-methylpentyl)-8-[(2,6-difluorobenzyl)oxy]-2,6-dimethylimidazo[1,2-a]pyridine-3-carboxamide (enantiomer A)
- *ent*-N-(2-amino-3-fluoro-2-methylpropyl)-2,6-dimethyl-8-[(2,3,6-trifluorobenzyl)oxy]imidazo[1,2-a]pyridine-3-carboxamide (enantiomer B)
- *ent*-N-(2-amino-3-fluoro-2-methylpropyl)-8-[(2,6-difluorobenzyl)oxy]-2,6-dimethylimidazo[1,2-a]pyridine-3-carboxamide (enantiomer B)
- *ent*-N-(2-amino-3-fluoro-2-methylpropyl)-8-[(2,6-difluorobenzyl)oxy]-2,6-dimethylimidazo[1,2-a]pyridine-3-carboxamide (enantiomer A)
- *rac*-N-(2-amino-3-fluoro-2-methylpropyl)-8-[(2,6-difluorobenzyl)oxy]-2,6-dimethylimidazo[1,2-a]pyridine-3-carboxamide formate
- *ent*-N-(2-amino-3-fluoro-2-methylpropyl)-2,6-dimethyl-8-[(2,3,6-trifluorobenzyl)oxy]imidazo[1,2-a]pyridine-3-carboxamide (enantiomer A)
- *ent*-N-(2-amino-3-fluoro-2-methylpropyl)-8-[(2,6-difluorobenzyl)oxy]-6-(difluoromethyl)-2-methylimidazo[1,2-a]pyridine-3-carboxamide (enantiomer B)
- *ent*-N-(2-amino-3-fluoro-2-methylpropyl)-8-[(2,6-difluorobenzyl)oxy]-6-(difluoromethyl)-2-methylimidazo[1,2-a]pyridine-3-carboxamide (enantiomer A)
- *ent*-N-(2-amino-3-fluoro-2-methylpropyl)-8-[(2,6-difluorobenzyl)oxy]-6-(fluoromethyl)-2-methylimidazo[1,2-a]pyridine-3 -carboxamide
- 1,1,1,3,3,3-Hexafluoro-2-[({5-fluoro-2-[1-(2-fluorobenzyl)-5-(1,2-oxazol-3-yl)-1H-pyrazol-3-yl]-4-pyrimidinyl}amino)methyl]-2-propanol (Praliciguat)
- 5-fluoro-2-[1-(2-fluorobenzyl)-5-(1,2-oxazol-3-yl)-1H-pyrazol-3-yl]pyrimidin-4-ol (IWP-051) and
- IWP-121, IWP-427, IWP-953, IW-1701, IW-6463

According to a further embodiment of the present invention, the sGC stimulator for use according to the invention is selected from the group consisting of:
- 2-[1-(2-fluorobenzyl)-1H-pyrazolo[3,4-b]pyridin-3-yl]-5-(4-morpholinyl)-4,6-pyrimidinediamine
- 2-[1-(2-fluorobenzyl)-1H-pyrazolo[3,4-b]pyridin-3-yl]-5-(4-pyridinyl)-4-pyrimidineamine
- methyl 4,6-diamino-2-[1-(2-fluorobenzyl)-1H-pyrazolo[3,4-b]pyridin-3-yl]-5-pyrimidinyl(methyl)carbamate (Riociguat)
- methyl 4,6-diamino-2-[1-(2-fluorobenzyl)-1H-pyrazolo[3,4-b]pyridin-3-yl]-5-pyrimidinylcarbamate (Nelociguat)
- methyl {4,6-diamino-2-[5-fluoro-1-(2-fluorobenzyl)-1H-pyrazolo[3,4-b]pyridine-3-yl]pyrimidin-5-yl}carbamate (Vericiguat)
- methyl {4,6-diamino-2-[5-fluoro-1-(2-fluorobenzyl)-1H-pyrazolo[3,4-b]pyridine-3-yl]pyrimidin-5-yl}methylcarbamate
- methyl {4,6-diamino-2-[5-fluoro-1-(2-fluorobenzyl)-1H-pyrazolo[3,4-b]pyridin-3-yl]pyrimidin-5-yl}(2,2,2-trifluoroethyl)carbamate
- 4-amino-2-[5-chloro-3(3,3,3-trifluoropropyl)-1H-indazol-1-yl]-5,5-dimethyl-5,7-dihydro-6H-pyrrolo [2,3 -d]pyrimidin-6-one
- 4-amino-2[5-chloro-3-(2,3,6-trifluorobenzyl)-1H-indazol-1-yl]-5,5-dimethyl-5,7-dihydro-6H-pyrrolo [2,3 -d]pyrimidin-6-one
- 4-amino-5,5-dimethyl-2-[3-(2,3,6-trifluorobenzyl)1H-thieno[3,4-c]pyrazol-1-yl]-5,7-dihydro-6H-pyrrolo[2,3-d]pyrimidin-6-one
- 4-amino-5,5-dimethyl-2-[3-(2,3,6-trifluorobenzyl)-1H-thieno[2,3-d]pyrazol-1-yl]-5,5-dimethyl-5,7-dihydro-6H-pyrrolo[2,3-d]pyrimidin-6-one
- 4-amino-5,5-dimethyl-2-[7-(2,3,6-trifluorobenzyl)imidazo[1,5-b]pyridazin-5-yl]-5,7-dihydro-6H-pyrrolo[2,3-d]pyrimidin-6-one
- 4-amino-2-[6-chloro-3-(2,3,6-trifluorobenzyl)imidazo[1,5-a]pyridin-1-yl]]-5,5-dimethyl-5,7-dihydro-6H-pyrrolo[2,3-d]pyrimidin-6-one
- 4-amino-2-[6-fluoro-3-(2,3,6-trifluorobenzyl)imidazo[1,5-a]pyridin-1-yl]]-5,5-dimethyl-5,7-dihydro-6H-pyrrolo[2,3-d]pyrimidin-6-one
- 4-amino-2-[6-fluoro-3-(2,3,6-trifluorobenzyl)6-fluoroimidazo[1,5-a]pyridin-1-yl]-5,5-dimethyl-5,7-dihydro-6H-pyrrolo[2,3-d]pyrimidin-6-one
- 4-amino-5,5-dimethyl-2-[3-(2,4,6-trifluorobenzyl)imidazo[1,5-a]pyridin-1-yl]]-5,7-dihydro-6H-pyrrolo[2,3-d]pyrimidin-6-one
- 4-amino-2-[3-(2-cyclopentylethyl)imidazo[1,5-a]pyridin-1-yl]-5,5-dimethyl-5,7-dihydro-6H-pyrrolo[2,3-d]pyrimidin-6-one
- 3-(4-amino-5-cyclopropylpyrimidin-2-yl)-1-(2-fluorobenzyl)-1*H*-pyrazolo[3,4-*b*]pyridine (BAY 41-2272)
- 2-{5-fluoro-1-[(3-fluoropyridin-2-yl)methyl]-1H-pyrazolo[3,4-b]pyridin-3-yl}-5-methyl-5-(trifluoromethyl)-4-[(3,3,3-trifluoropropyl)amino]-5,7-dihydro-6H-pyrrolo[2,3-d]pyrimidin-6-one and
- 1,1,1,3,3,3-Hexafluoro-2-[({5-fluoro-2-[1-(2-fluorobenzyl)-5-(1,2-oxazol-3-yl)-1H-pyrazol-3-yl]-4-pyrimidinyl}amino)methyl]-2-propanol (Praliciguat)

According to a further embodiment of the present invention, the sGC stimulator for use according to the invention is selected from the group consisting of:
- 2-[1-(2-fluorobenzyl)-1H-pyrazolo[3,4-b]pyridin-3-yl]-5-(4-morpholinyl)-4,6-pyrimidinediamine
- 2-[1-(2-fluorobenzyl)-1H-pyrazolo[3,4-b]pyridin-3-yl]-5-(4-pyridinyl)-4-pyrimidineamine
- methyl 4,6-diamino-2-[1-(2-fluorobenzyl)-1H-pyrazolo[3,4-b]pyridin-3-yl]-5-pyrimidinyl(methyl)carbamate (Riociguat)
- methyl 4,6-diamino-2-[1-(2-fluorobenzyl)-1H-pyrazolo[3,4-b]pyridin-3-yl]-5-pyrimidinylcarbamate (Nelociguat)
- methyl {4,6-diamino-2-[5-fluoro-1-(2-fluorobenzyl)-1H-pyrazolo[3,4-b]pyridine-3-yl]pyrimidin-5-yl}carbamate (Vericiguat)
- methyl {4,6-diamino-2-[5-fluoro-1-(2-fluorobenzyl)-1H-pyrazolo[3,4-b]pyridine-3-yl]pyrimidin-5-yl}methylcarbamate
- 3-(4-amino-5-cyclopropylpyrimidin-2-yl)-1-(2-fluorobenzyl)-1*H*-pyrazolo[3,4-*b*]pyridine (BAY 41-2272)
- 2-{5-fluoro-1-[(3-fluoropyridin-2-yl)methyl]-1H-pyrazolo[3,4-b]pyridin-3-yl}-5-methyl-5-(trifluoromethyl)-4-[(3,3,3-trifluoropropyl)amino]-5,7-dihydro-6H-pyrrolo[2,3-d]pyrimidin-6-one and
- 1,1,1,3,3,3-Hexafluoro-2-[({5-fluoro-2-[1-(2-fluorobenzyl)-5-(1,2-oxazol-3-yl)-1H-pyrazol-3-yl]-4-pyrimidinyl}amino)methyl]-2-propanol (Praliciguat)

According to a further embodiment of the present invention, the sGC stimulator for use according to the invention is selected from the group consisting of:
- methyl 4,6-diamino-2-[1-(2-fluorobenzyl)-1H-pyrazolo[3,4-b]pyridin-3-yl]-5-pyrimidinyl(methyl)carbamate (Riociguat)
- methyl 4,6-diamino-2-[1-(2-fluorobenzyl)-1H-pyrazolo[3,4-b]pyridin-3-yl]-5-pyrimidinylcarbamate (Nelociguat)
- methyl {4,6-diamino-2-[5-fluoro-1-(2-fluorobenzyl)-1H-pyrazolo[3,4-b]pyridine-3-yl]pyrimidin-5-yl}carbamate (Vericiguat)
- methyl {4,6-diamino-2-[5-fluoro-1-(2-fluorobenzyl)-1H-pyrazolo[3,4-b]pyridine-3-yl]pyrimidin-5-yl}methylcarbamate
- 2-{5-fluoro-1-[(3-fluoropyridin-2-yl)methyl]-1H-pyrazolo[3,4-b]pyridin-3-yl}-5-methyl-5-(trifluoromethyl)-4-[(3,3,3-trifluoropropyl)amino]-5,7-dihydro-6H-pyrrolo[2,3-d]pyrimidin-6-one and
- 3-(4-amino-5-cyclopropylpyrimidin-2-yl)-1-(2-fluorobenzyl)-1*H*-pyrazolo[3,4-*b*]pyridine (BAY 41-2272)
- 1,1,1,3,3,3-Hexafluoro-2-[({5-fluoro-2-[1-(2-fluorobenzyl)-5-(1,2-oxazol-3-yl)-1H-pyrazol-3-yl]-4-pyrimidinyl}amino)methyl]-2-propanol (Praliciguat)

According to a further embodiment of the present invention, the sGC stimulator for use according to the invention is selected from the group consisting of:
- methyl 4,6-diamino-2-[1-(2-fluorobenzyl)-1H-pyrazolo[3,4-b]pyridin-3-yl]-5-pyrimidinyl(methyl)carbamate (Riociguat)
- methyl {4,6-diamino-2-[5-fluoro-1-(2-fluorobenzyl)-1H-pyrazolo[3,4-b]pyridine-3-yl]pyrimidin-5-yl}carbamate (Vericiguat) and
- 3-(4-amino-5-cyclopropylpyrimidin-2-yl)-1-(2-fluorobenzyl)-1*H*-pyrazolo[3,4-*b*]pyridine (BAY 41-2272)
- 1,1,1,3,3,3-Hexafluoro-2-[({5-fluoro-2-[1-(2-fluorobenzyl)-5-(1,2-oxazol-3-yl)-1H-pyrazol-3-yl]-4-pyrimidinyl}amino)methyl]-2-propanol (Praliciguat)

According to a further embodiment of the present invention, the sGC stimulator for use according to the invention is:
- methyl {4,6-diamino-2-[5-fluoro-1-(2-fluorobenzyl)-1H-pyrazolo[3,4-b]pyridine-3-yl]pyrimidin-5-yl}carbamate (Vericiguat)

According to a further embodiment of the present invention, the sGC stimulator for use according to the invention is:
- methyl 4,6-diamino-2-[1-(2-fluorobenzyl)-1H-pyrazolo[3,4-b]pyridin-3-yl]-5-pyrimidinyl(methyl)carbamate (Riociguat)

According to a further embodiment of the present invention, the sGC stimulator for use according to the invention is:
- 3-(4-amino-5-cyclopropylpyrimidin-2-yl)-1-(2-fluorobenzyl)-1*H*-pyrazolo[3,4-*b*]pyridine (BAY 41-2272)

According to a further embodiment of the present invention, the sGC stimulator for use according to the invention is:
- 1,1,1,3,3,3-Hexafluoro-2-[({5-fluoro-2-[1-(2-fluorobenzyl)-5-(1,2-oxazol-3-yl)-1H-pyrazol-3-yl]-4-pyrimidinyl}amino)methyl]-2-propanol (Praliciguat)

The present invention provides sGC activators for use in the treatment and/or prophylaxis of bone diseases, wherein the sGC activator is selected from the group consisting of:
- 4-({(4-carboxybutyl)[2-(2-{[4-(2-phenylethyl)benzyl]oxy}phenyl)ethyl]amino}methyl)benzoic acid
- 5-chloro-2-(5-chlorothiophene-2-sulfonylamino-N-(4-(morpholine-4-sulfonyl)phenyl)benzamide as sodium salt
- 2-(4-chlorophenylsulfonylamino)-4,5-dimethoxy-N-(4-(thiomorpholine-4-sulfonyl)phenyl)benzamide
- 1-{6-[5-chloro-2-({4-trans-4-}trifluoromethyl)cyclohexyl]benzyl}oxy)phenyl]pyridin-2-yl}-5-(trifluoromethyl)-1H-pyrazole-4-carboxylic acid
- 1-[6-(2-(2-methyl-4-(4-trifluoromethoxyphenyl)benzyloxy)phenyl)pyridin-2-yl]-5-trifluoromethylpyrazole-4-carboxylic acid
- 1[6-(3,4-dichlorophenyl)-2-pyridinyl-5-(trifluoromethyl)-1H-pyrazole-4-carboxylic acid
- 1-({2-[3-chloro-5-(trifluoromethyl)phenyl]-5-methyl-1,3-thiazol-4-yl}methyl)-1H-pyrazole-4-carboxylic acid
- 4-({2-[3-(trifluoromethyl)phenyl]-1,3-thiazol-4-yl}methyl)benzoic acid
- 1-({2-[2-fluoro-3-(trifluoromethyl)phenyl]-5-methyl-1,3-thiazol-4-yl}methyl)-1H-pyrazole-4-carboxylic acid
- 3-(4-chloro-3-{[(2S,3R)-2-(4-chlorophenyl)-4,4,4-trifluoro-3-methylbutanoyl]amino}phenyl)-3-cyclopropylpropanoic acid
- 5-{[2-(4-carboxyphenyl)ethyl][2-(2-{[3-chloro-4'-(trifluoromethyl)biphenyl-4-yl]methoxy}phenyl)ethyl]amino}-5,6,7,8-tetrahydroquinoline-2-carboxylic acid
- 5-{(4-carboxybutyl)[2-(2-{[3-chloro-4'-(trifluoromethyl)biphenyl-4-yl]methoxy}phenyl)ethyl]amino}-5,6,7,8-tetrahydroquinoline-2-carboxylic acid
- (1R,5S)-3-[4-(5-methyl-2-{[2-methyl-4-(piperidin-1-ylcarbonyl)benzyl]oxy}phenyl)-1,3-thiazol-2-yl]-3-azabicyclo[3.2.1]octane-8-carboxylic acid and
- 1-[6-(5-methyl-2-{[2-(tetrahydro-2H-pyran-4-yl)-1,2,3,4-tetrahydroisoquinolin-6-yl]methoxy}phenyl)pyridin-2-yl]-5-(trifluoromethyl)-1H-pyrazole-4-carboxylic acid

According to a further embodiment of the present invention, the sGC activator for use according to the invention is selected from the group consisting of::
- 3-(4-chloro-3-{[(2S,3R)-2-(4-chlorophenyl)-4,4,4-trifluoro-3-methylbutanoyl] amino}phenyl)-3-cyclopropylpropanoic acid
- 5-{[2-(4-carboxyphenyl)ethyl][2-(2-{[3-chloro-4'-(trifluoromethyl)biphenyl-4-yl]methoxy}phenyl)ethyl]amino}-5,6,7,8-tetrahydroquinoline-2-carboxylic acid and
- 5-{(4-carboxybutyl)[2-(2-{[3-chloro-4'-(trifluoromethyl)biphenyl-4-yl]methoxy}phenyl)ethyl]amino}-5,6,7,8-tetrahydroquinoline-2-carboxylic acid

According to a further embodiment of the present invention, the sGC activator for use according to the invention is:
- 3-(4-chloro-3-{[(2S,3R)-2-(4-chlorophenyl)-4,4,4-trifluoro-3-methylbutanoyl] amino}phenyl)-3-cyclopropylpropanoic acid

Further sGC activators in the context of the invention are known from the following publications: WO2013/157528, WO2015/056663, WO2009/123316, WO2016/001875, WO2016/001876, WO2016/001878, WO2000/02851, WO2012/122340, WO2013/025425, WO2014/039434, WO2016/014463, WO2009/071504, WO2010/015652, WO2010/015653, WO2015/033307, WO2016/042536, WO2009/032249, WO2010/099054, WO2012/058132, US2010/0216764, WO2001/19776, WO2001/19780, WO2001/19778, WO2002/070459, WO2002/070460, WO2002/070510, WO2002/070462, WO2007/045366, WO2007/045369, WO2007/045433, WO2007/045370, WO2007/045367, WO2014/012935, WO2014/012934, WO2011/141409, WO2008/119457, WO2008/119458, WO2009/127338, WO2010/102717, WO2011/051165, WO2012/076466, WO2012/139888, WO2013/157528, WO2013/174736, WO2014/012934, WO2015/056663, WO2017103888, WO2017112617, WO2016042536, WO2016081668, WO2016191335, WO2016191334, WO2016001875, WO2016001876, WO2016001878, WO2016014463, WO2016044447, WO2016044445, WO2016044446, WO2015056663, WO2015033307, WO2015187470, WO2015088885, WO2015088886, WO2015089182, WO2014084312, WO2014039434, WO2014144100, WO2014047111, WO2014047325, WO2013025425, WO2013101830, WO2012165399, WO2012058132, WO2012122340, WO2012003405, WO2012064559, WO2011149921, WO2011119518, WO2011115804, WO2011056511, CN101670106, TW201028152, WO2010015653, WO2010015652, WO2010099054, WO2010065275, WO2009123316, WO2009068652, WO2009071504, WO2009032249, US2009209556.

The present invention further provides PDE5 inhibitors for use in the treatment *and*/*or prophylaxis* of bone diseases. According to an embodiment of the present invention, the PDE5 inhibitor is selected from the group consisting of:
- Tadalafil ((6R,12aR) -2,3,6,7,12,12a - Hexahydro - 2 - methyl - 6 - (3,4-methylene -dioxyphenyl) pyrazino(1',2':1,6) pyrido(3,4-b)indole-1,4-dione)
- Vardenafil (2-(2-Ethoxy-5-(4-ethylpiperazin-1-yl-1-sulfonyl)phenyl)-5-methyl-7-propyl-3H-imidazo (5,1-f)(1,2,4)triazin-4-one)
- Sildenafil (3-[2-ethoxy-5-(4-methylpiperazin-1-yl)sulfonyl-phenyl]-7-methy1-9-propy1-2,4,7,8-tetrazabicyclo [4.3.0]nona -3,8,10-trien-5-one)
- Udenafil 5-[2-propyloxy-5-(1-methyl-2-pyrrolidinylethylamidosulfonyl)phenyl]-methyl-3-propyl-1,6-dihydro-7H-pyrazolo(4,3-d)pyrimidine-7-one
- Dasantafil 7-(3-Bromo-4-methoxybenzyl)-1-ethyl-8-[[(1,2)-2-hydroxycyclopentyl]amino]-3-(2-hydroxyethyl)-3,7-dihydro-1-purine-2,6-dione
- Avanafil 4-{[(3-chloro-4-methoxyphenyl)methyl]amino}-2-[(2S)-2-(hydroxymethyl)pyrrolidin-1-yl]-N-(pyrimidin-2-ylmethyl)pyrimidine-5-carboxamide
- Thioquinapiperifil
- Zaprinast
- Mirodenafil
- Lodenafil
- UK 369003, UK 371800, UK 114502, UK 114542
- SLx 2101
- LAS 34179 Triazolo[1,2-]xanthine,6-methyl-4-propyl-2-[2-propoxy-5-(4-methylpiperazino)sulfonyl]phenyl
- LAS 30904, LAS 34837
- BMS 341400, BMS 281384, BMS 263504,
- AWD 12-250
- FR 189318, FR 229934, FR 226807,
- EMD 82639
- EMR 62-203
- QAD 171A and
- SR 265579

The sGC stimulators and sGC activators according to the invention can be used alone or, if required, in combination with other active ingredients. The present invention further provides medicaments comprising at least one of the compounds of the invention and one or more further active compounds, especially for the treatment and/or prophylaxis of the aforementioned disorders. Preferred examples of suitable combination active ingredients include:
- compounds which inhibit the breakdown of cyclic guanosine monophosphate (cGMP), such as, for example, inhibitors of phosphodiesterases (PDE) 1, 2 and/or 5, in particular PDE 5 inhibitors such as sildenafil, vardenafil and tadalafil;
- calcium
- vitamin D and metabolites of vitamin D, for example calcitriol
- Bisphosphonates, for example Etidronate, Clodronate, Tiludronate, Teriparatide, Pamidronate, Neridronate, Olpadronate, Alendronate, Ibandronate, Risedronate, Zoledronate
- strontium ranelate
- active ingredients suitable for hormone replacement therapy in osteoporosis, for example estrogen or a combination of estrogen and progesterone
- selective estrogen receptor modulators (SERMs), for example raloxifene
- parathyroid hormone or analogs of parathyroid hormone, for example teriparatide
- modulators of receptor activator of nuclear factor kappa-B ligand (RANKL), for example denosumab
- Sclerostin inhibitors, for example romosozumab or BPS804
- TGF-β inhibitors, for example fresolimumab

The present invention further provides sGC stimulators and sGC activators for use in the treatment and/or prophylaxis of osteoporosis, primary osteoporosis, and secondary osteoporosis.

The present invention further provides sGC stimulators and sGC activators for use in the treatment and/or prophylaxis of osteoporosis, primary osteoporosis, and secondary osteoporosis, wherein the osteoporosis, primary osteoporosis, or secondary osteoporosis is not caused by a deficiency of sex hormones.

According to a further embodiment of the present invention the sGC stimulator for use in the treatment and/or prophylaxis of bone diseases selected from the group consisting of osteogenesis imperfecta (OI), bone fractures, impaired bone healing, rickets, osteomalacia, avascular bone necrosis, and Paget disease is selected from the group consisting of
- methyl 4,6-diamino-2-[1-(2-fluorobenzyl)-1H-pyrazolo[3,4-b]pyridin-3-yl]-5-pyrimidinyl(methyl)carbamate (Riociguat)
- methyl {4,6-diamino-2-[5-fluoro-1-(2-fluorobenzyl)-1H-pyrazolo[3,4-b]pyridine-3-yl]pyrimidin-5-yl}carbamate (Vericiguat) and
- 3-(4-amino-5-cyclopropylpyrimidin-2-yl)-1-(2-fluorobenzyl)-1*H*-pyrazolo[3,4-*b*]pyridine (BAY 41-2272) and
- 1,1,1,3,3,3-Hexafluoro-2-[({5-fluoro-2-[1-(2-fluorobenzyl)-5-(1,2-oxazol-3-yl)-1H-pyrazol-3-yl]-4-pyrimidinyl}amino)methyl]-2-propanol (Praliciguat).

According to a further embodiment of the present invention the sGC stimulator for use in the treatment and/or prophylaxis of osteogenesis imperfecta (OI) is selected from the group consisting of
- methyl 4,6-diamino-2-[1-(2-fluorobenzyl)-1H-pyrazolo[3,4-b]pyridin-3-yl]-5-pyrimidinyl(methyl)carbamate (Riociguat)
- methyl {4,6-diamino-2-[5-fluoro-1-(2-fluorobenzyl)-1H-pyrazolo[3,4-b]pyridine-3-yl]pyrimidin-5-yl}carbamate (Vericiguat) and
- 3-(4-amino-5-cyclopropylpyrimidin-2-yl)-1-(2-fluorobenzyl)-1*H*-pyrazolo[3,4-*b*]pyridine (BAY 41-2272) and
- 1,1,1,3,3,3-Hexafluoro-2-[({5-fluoro-2-[1-(2-fluorobenzyl)-5-(1,2-oxazol-3-yl)-1H-pyrazol-3-yl]-4-pyrimidinyl}amino)methyl]-2-propanol (Praliciguat).

According to a further embodiment of the present invention the sGC stimulator for use in the treatment and/or prophylaxis of bone diseases selected from the group consisting of bone fractures and impaired bone healing, is selected from the group consisting of
- methyl 4,6-diamino-2-[1-(2-fluorobenzyl)-1H-pyrazolo[3,4-b]pyridin-3-yl]-5-pyrimidinyl(methyl)carbamate (Riociguat)
- methyl {4,6-diamino-2-[5-fluoro-1-(2-fluorobenzyl)-1H-pyrazolo[3,4-b]pyridine-3-yl]pyrimidin-5-yl}carbamate (Vericiguat) and
- 3-(4-amino-5-cyclopropylpyrimidin-2-yl)-1-(2-fluorobenzyl)-1*H*-pyrazolo[3,4-*b*]pyridine (BAY 41-2272) and
- 1,1,1,3,3,3-Hexafluoro-2-[({5-fluoro-2-[1-(2-fluorobenzyl)-5-(1,2-oxazol-3-yl)-1H-pyrazol-3-yl]-4-pyrimidinyl}amino)methyl]-2-propanol (Praliciguat).

According to a further embodiment of the present invention the sGC stimulator for use in the treatment and/or prophylaxis of bone diseases selected from the group consisting of rickets, osteomalacia, avascular bone necrosis, and Paget disease is selected from the group consisting of
- methyl 4,6-diamino-2-[1-(2-fluorobenzyl)-1H-pyrazolo[3,4-b]pyridin-3-yl]-5-pyrimidinyl(methyl)carbamate (Riociguat)
- methyl {4,6-diamino-2-[5-fluoro-1-(2-fluorobenzyl)-1H-pyrazolo[3,4-b]pyridine-3-yl]pyrimidin-5-yl}carbamate (Vericiguat) and
- 3-(4-amino-5-cyclopropylpyrimidin-2-yl)-1-(2-fluorobenzyl)-1*H*-pyrazolo[3,4-*b*]pyridine (BAY 41-2272) and
- 1,1,1,3,3,3-Hexafluoro-2-[({5-fluoro-2-[1-(2-fluorobenzyl)-5-(1,2-oxazol-3-yl)-1H-pyrazol-3-yl]-4-pyrimidinyl}amino)methyl]-2-propanol (Praliciguat).

According to a further embodiment of the present invention the sGC stimulator for use in the treatment and/or prophylaxis of bone diseases selected from the group consisting of osteoporosis, primary osteoporosis, and secondary osteoporosis is selected from the group consisting of
- methyl 4,6-diamino-2-[1-(2-fluorobenzyl)-1H-pyrazolo[3,4-b]pyridin-3-yl]-5-pyrimidinyl(methyl)carbamate (Riociguat)
- methyl {4,6-diamino-2-[5-fluoro-1-(2-fluorobenzyl)-1H-pyrazolo[3,4-b]pyridine-3-yl]pyrimidin-5-yl}carbamate (Vericiguat) and
- 3-(4-amino-5-cyclopropylpyrimidin-2-yl)-1-(2-fluorobenzyl)-1*H*-pyrazolo[3,4-*b*]pyridine (BAY 41-2272) and
- 1,1,1,3,3,3-Hexafluoro-2-[({5-fluoro-2-[1-(2-fluorobenzyl)-5-(1,2-oxazol-3-yl)-1H-pyrazol-3-yl]-4-pyrimidinyl}amino)methyl]-2-propanol (Praliciguat).

According to a further embodiment of the present invention the sGC stimulator for use in the treatment and/or prophylaxis of bone diseases selected from the group consisting of osteoporosis, primary osteoporosis, and secondary osteoporosis, wherein the osteoporosis, primary osteoporosis, and secondary osteoporosis is not caused by a deficiency of sex hormones, is selected from the group consisting of
- methyl 4,6-diamino-2-[1-(2-fluorobenzyl)-1H-pyrazolo[3,4-b]pyridin-3-yl]-5-pyrimidinyl(methyl)carbamate (Riociguat)
- methyl {4,6-diamino-2-[5-fluoro-1-(2-fluorobenzyl)-1H-pyrazolo[3,4-b]pyridine-3-yl]pyrimidin-5-yl}carbamate (Vericiguat) and
- 3-(4-amino-5-cyclopropylpyrimidin-2-yl)-1-(2-fluorobenzyl)-1*H*-pyrazolo[3,4-*b*]pyridine (BAY 41-2272) and
- 1,1,1,3,3,3-Hexafluoro-2-[({5-fluoro-2-[1-(2-fluorobenzyl)-5-(1,2-oxazol-3-yl)-1H-pyrazol-3-yl]-4-pyrimidinyl}amino)methyl]-2-propanol (Praliciguat).

According to a further embodiment of the present invention the sGC activator for use in the treatment and/or prophylaxis of bone diseases selected from the group consisting of osteogenesis imperfecta (OI), bone fractures, impaired bone healing, rickets, osteomalacia, avascular bone necrosis, and Paget disease is 3-(4-chloro-3-{[(2S,3R)-2-(4-chlorophenyl)-4,4,4-trifluoro-3-methylbutanoyl]amino}phenyl)-3-cyclopropylpropanoic acid.

According to a further embodiment of the present invention the sGC activator for use in the treatment and/or prophylaxis of osteogenesis imperfecta (OI) is 3-(4-chloro-3-{[(2S,3R)-2-(4-chlorophenyl)-4,4,4-trifluoro-3 -methylbutanoyl] amino}phenyl)-3 -cyclopropylpropanoic acid.

According to a further embodiment of the present invention the sGC activator for use in the treatment and/or prophylaxis of bone diseases selected from the group consisting of bone fractures and impaired bone healing, is 3-(4-chloro-3-{[(2S,3R)-2-(4-chlorophenyl)-4,4,4-trifluoro-3-methylbutanoyl]amino}phenyl)-3-cyclopropylpropanoic acid.

According to a further embodiment of the present invention the sGC activator for use in the treatment and/or prophylaxis of bone diseases selected from the group consisting of rickets, osteomalacia, avascular bone necrosis, and Paget disease is 3-(4-chloro-3-{[(2S,3R)-2-(4-chlorophenyl)-4,4,4-trifluoro-3-methylbutanoyl] amino}phenyl)-3 -cyclopropylpropanoic acid.

According to a further embodiment of the present invention the sGC activator for use in the treatment and/or prophylaxis of bone diseases selected from the group consisting of osteoporosis, primary osteoporosis, and secondary osteoporosis is 3-(4-chloro-3-{[(2S,3R)-2-(4-chlorophenyl)-4,4,4-trifluoro-3-methylbutanoyl] amino}phenyl)-3 -cyclopropylpropanoic acid.

According to a further embodiment of the present invention the sGC activator for use in the treatment and/or prophylaxis of bone diseases selected from the group consisting of osteoporosis, primary osteoporosis, and secondary osteoporosis, wherein the osteoporosis, primary osteoporosis, and secondary osteoporosis is not caused by a deficiency of sex hormones, is 3-(4-chloro-3-{[(2S,3R)-2-(4-chlorophenyl)-4,4,4-trifluoro-3-methylbutanoyl] amino}phenyl) -3 -cyclopropylpropanoic acid.

The present invention further provides pharmaceutical compositions which comprise at least one sGC stimulator or sGC activator, typically together with one or more inert, nontoxic, pharmaceutically suitable excipients, and for the use thereof for the aforementioned purposes. This can be accomplished in a manner known per se by mixing with inert, nontoxic, pharmaceutically suitable excipients. These excipients include carriers (for example microcrystalline cellulose, lactose, mannitol), solvents (e.g. liquid polyethylene glycols), emulsifiers and dispersing or wetting agents (for example sodium dodecylsulphate, polyoxysorbitan oleate), binders (for example polyvinylpyrrolidone), synthetic and natural polymers (for example albumin), stabilizers (e.g. antioxidants, for example ascorbic acid), colorants (e.g. inorganic pigments, for example iron oxides) and flavour and/or odour correctants.

Another embodiment of the invention is a pharmaceutical composition comprising at least one sGC stimulator or sGC activator in combination with one or more inert non-toxic pharmaceutically suitable excipients for use in the treatment and/or prophylaxis of bone diseases selected from the group consisting of osteogenesis imperfecta (OI), bone fractures, impaired bone healing, rickets, osteomalacia, avascular bone necrosis, and Paget disease.

Another embodiment of the invention is a pharmaceutical composition comprising at least one sGC stimulator or sGC activator in combination with one or more inert non-toxic pharmaceutically suitable excipients for use in the treatment and/or prophylaxis of bone diseases selected from the group consisting of osteoporosis, primary osteoporosis, and secondary osteoporosis, wherein the osteoporosis, primary osteoporosis, and secondary osteoporosis is not caused by a deficiency of sex hormones.

Another embodiment of the invention is a pharmaceutical composition comprising at least one sGC stimulator or sGC activator and at least one compound selected from the group consisting of inhibitors of phosphodiesterases (PDE) 1, 2 and/or 5, calcium, vitamin D and metabolites of vitamin D, bisphosphonates, selected from etidronate, clodronate, tiludronate, teriparatide, pamidronate, neridronate, olpadronate, alendronate, ibandronate, risedronate, and zoledronate, strontium ranelate, active ingredients suitable for hormone replacement therapy in osteoporosis, selected from estrogen and a combination of estrogen and progesterone, selective estrogen receptor modulators (SERMs), parathyroid hormone and analogs of parathyroid hormone, modulators of receptor activator of nuclear factor kappa-B ligand (RANKL), sclerostin inhibitors, and TGF-β inhibitors for use in the treatment and/or prophylaxis of bone diseases selected from the group consisting of osteogenesis imperfecta (OI), bone fractures, impaired bone healing, rickets, osteomalacia, avascular bone necrosis, and Paget disease.

Another embodiment of the invention is a pharmaceutical composition comprising at least one sGC stimulator or sGC activator and at least one compound selected from the group consisting of inhibitors of phosphodiesterases (PDE) 1, 2 and/or 5, calcium, vitamin D and metabolites of vitamin D, bisphosphonates, selected from etidronate, clodronate, tiludronate, teriparatide, pamidronate, neridronate, olpadronate, alendronate, ibandronate, risedronate, and zoledronate, strontium ranelate, active ingredients suitable for hormone replacement therapy in osteoporosis, selected from estrogen and a combination of estrogen and progesterone, selective estrogen receptor modulators (SERMs), parathyroid hormone and analogs of parathyroid hormone, modulators of receptor activator of nuclear factor kappa-B ligand (RANKL), sclerostin inhibitors, and TGF-β inhibitors for use in the treatment and/or prophylaxis of bone diseases selected from the group consisting of osteoporosis, primary osteoporosis, and secondary osteoporosis, wherein the osteoporosis, primary osteoporosis, and secondary osteoporosis is not caused by a deficiency of sex hormones.

Another embodiment of the invention is a pharmaceutical composition comprising at least one sGC stimulator or sGC activator and at least one inhibitor of phosphodiesterase (PDE) 5 selected from the group consisting of sildenafil, vardenafil, tadalafil and avanafil for use in the treatment and/or prophylaxis of bone diseases selected from the group consisting of osteogenesis imperfecta (OI), bone fractures, impaired bone healing, rickets, osteomalacia, avascular bone necrosis, and Paget disease.

Another embodiment of the invention is a pharmaceutical composition comprising at least one sGC stimulator or sGC activator and at least one inhibitor of phosphodiesterase (PDE) 5 selected from the group consisting of sildenafil, vardenafil, tadalafil and avanafil for use in the treatment and/or prophylaxis of bone diseases selected from the group consisting of osteoporosis, primary osteoporosis, and secondary osteoporosis, wherein the osteoporosis, primary osteoporosis, and secondary osteoporosis is not caused by a deficiency of sex hormones.

### Investigation of therapeutic efficacy:

For investigations of the effects of sGC stimulators and PDE5i as stand-alone treatment or in combination, a broad spectrum of in vitro, ex vivo and in vivo tests were used.

Preferentially mice were used, especially transgenic mice (B6C3Fe a/a-Col1a2 oim /J (Jackson Laboratories)) (Chipman et al. 1993, McBride et al. 1998). These so called OI-mice (OIM, also called HOM mice) carry a mutation in the collagen gen (Col1a2) which reflects the phenotype and clinical situation of patients with muscular OI. For the treatment homozygous OIM are used and compared with WT mice which serve as control.

As read-outs were preferentially used:
- semiquantitative RT/PCR (TaqMan PCR) to assess gene expression according to standard procedures
- histopathology to assess bone morphology according to standard procedures
- high-resolution micro-CTscanner (µCT 35; Scanco Medical AG, Briittisellen, Switzerland) to evaluate bone morphology and architecture (Micro-CT)
- three-point bending test to assess mechanical properties of the femora (Mechanical testing)

### Micro-CT

A high-resolution micro-CTscanner (µCT 35; Scanco Medical AG, Briittisellen, Switzerland) was used to evaluate bone morphology and architecture. Femora were placed in a specimen tube filled with 0.9% (m/v) NaCl including protease inhibitors (Roche complete EDTA-free Protease Inhibitor Cocktail, Roche Diagnostics GmbH, Mannheim, Germany) and scanned with an isotropic voxel size of 7 µm using, 70 kVp tube voltage, 114 mA tube current, and 400 ms integration time. The distal femur was imaged at the metaphyseal trabecular region and at the diaphysis to analyse the cortical region. A 1 mm volume of interest was defined 1 mm below the growth plate for trabecular bone, while a region of 0.75 mm for cortical bone analysis was situated at 50 % of the total bone length. Gray-scale images were segmented using a constrained gaussian filter (trabecular: sigma = 0.8, support = 1; cortical: sigma = 0.8, support = 2.0) to reduce partly the noise in the original volume data. Image data were segmented using a threshold of 23 % of the maximum grey scale value for the trabecular compartment and 28 % for the cortical compartment. The trabecular and cortical parameters were determined according to the guidelines for the assessment of bone microstructure with micro-CT (Bouxsein et al., 2010). For trabecular bone analyses, bone volume (BV, mm³), tissue volume (TV, mm³), bone volume fraction (BV/TV, %), trabecular separation (Tb.Sp, µm), trabecular thickness (Tb.Th, µm), trabecular number (Tb.N, 1/mm), and connectivity density (Conn.D, 1/mm³) were determined. Cortical parameters included tissue area (Tt.Ar, mm²), cortical area (Ct.Ar, mm²), marrow area (Ma.Ar, mm²), cortical thickness (Ct.Th, mm), and cortical area fraction (Ct.Ar/Tt.Ar, %).

### Mechanical testing

Mechanical properties of the femora were tested using a materials testing machine (Z2.5/TN1S, Zwick GmbH & CoKG, Ulm, Germany). Bones were loaded at the mid-diaphysis in anterior-posterior direction until failure by a three-point bending test. The distance of the lower support points (Ø 1.5 mm) was 5 mm. The tests were conducted using a 100 N load cell. After pre-loading (0.1 N, 0.05 mm/s) the breaking force was applied with a crosshead speed of 1/mm. Ultimate force (N), deformation (mm) and energy (mJ) were obtained from the load-deformation curve. Ultimate stress (MPa) and strain were calculated considering the cross-sectional moments of inertia obtained from the µCT measurements. Stiffness (N/mm) and Young's modulus (GPa) were determined from the slope of the linear portion of the load-deformation and stress-strain curves, respectively (Turner et al., 1993).

### Examples:

A 16 week chronic treatment study was performed in male and female B6C3Fe a/a-Col1a2 oim /J (OI mice) termed as HOM (homozygous B6C3Fe a/a-Col1a2 oim /OI mice) and in male and female wild-type mice (WT). Both groups, HOM and WT mice, were randomized in two groups and received either placebo, or BAY 41-2272 at 300 ppm (300mg/kg chow), suspended in standard mouse chow. As standard mouse chow, the diet number V1530-000 from ssniff R/M-H (powder grade) was used and the compound was homogenously suspended at room temperature by using a mixing granulator manufactured by Loedige, Paderborn, Germany. Group size was n=16 mice in WT groups and n=6-10 in HOM groups).

The expression "ppm" (parts per million) describes a weight to weight ratio used to describe concentrations. In the present experiment "ppm" is the number of units of mass of BAY 41-2272 per million units of total mass of the food for feeding mice; food consumption was 2.5-5.0g/mouse/day.

There were statistically (P < 0.05) significant differences between female WT and HOM mice in trabecular number, trabecular space, tissue area, cortical thickness, and cortical area.

The treatment with 300 ppm had no effect on structural properties of both trabecular and cortical bone in WT mice. Interestingly, the treatment with 300 ppm increased trabecular number (Tb.N) and decreased trabecular separation (Tb.Sp) in treated female HOM mice compared to untreated female HOM mice (P < 0.05).

### References:

Bonafé L, Giunta C, Hasler C, Janner M, Kränzlin M, Link B, Meier C, Ramseier LE, Rohrbach M, Unger S. Osteogenesis imperfecta: Klinik, Diagnose und Management vom Kindes- bis ins Erwachsenenalter. Schweiz Med Forum 2013;13(46):925-931.
Bouxsein ML, Boyd SK, Christiansen BA, Guldberg RE, Jepsen KJ, Müller R (2010). Guidelines for assessment of bone microstructure in rodents using micro-computed tomography. J Bone Miner Res 25(7):1468-86
Chipman SD, Sweet HO, McBride DJ Jr, Davisson MT, Marks SC Jr, Shuldiner AR, Wenstrup RJ, Rowe DW, Shapiro JR. Defective pro alpha 2(1) collagen synthesis in a recessive mutation in mice: a model of human osteogenesis imperfecta. Proc Natl Acad Sci USA. 1993;90(5):1701-05
Evgenov OV, Pacher P, Schmidt PM, Haskó G, Schmidt HH, Stasch JP. NO-independent stimulators and activators of soluble guanylate cyclase: discovery and therapeutic potential. Nat Rev Drug Discov. 2006 Sep;5(9):755-68
FDA 2013, summary basis of approval, riociguat, pharmacology review, https://www.accessdata.fda.gov/drugsatfda docs/nda/2013/204819Orig1s000PharmR.pdf, pages 1-3 and 9-20 of the report.
Follmann M et al. Discovery of the Soluble Guanylate Cyclase Stimulator Vericiguat (BAY 1021189) for the Treatment of Chronic Heart Failure. J. Med Chem 2017 Jun 22;60(12):5146-5161
Histing T, Marciniak K, Scheuer C, et al. Sildenafil accelerates fracture healing in mice. J Orthop Res 2011;29:867-873.
Joshua J, Schwaerzer GK, Kalyanaraman H, Cory E, Sah RL, Li M, Vaida F, Boss GR, Pilz RB. Soluble guanylate cyclase as a novel treatment target for osteoporosis. Endocrinology. 2014 Dec;155(12):4720-30
McBride DJ Jr; Shapiro JR; Dunn MG. Bone geometry and strength measurements in aging mice with the oim mutation. Calcif Tissue Int 1998;62(2):172-6.
Medscape, https://emedicine.medscape.com/article/2089003-overview
Monti E, Mottes M, Fraschini P, Brunelli P, Forlino A, Venturi G, Doro F, Perlini S, Cavarzere P, Antoniazzi F. Current and emerging treatments for the management of osteogenesis imperfecta. Ther Clin Risk Manag. 2010 Sep 7;6:367-381.
Rajfer RA, Kilic A, Neviaser AS, Schulte LM, Hlaing SM, Landeros J, Ferrini MG, Ebramzadeh E, Park SH, Enhancement of fracture healing in the rat, modulated by compounds that stimulate inducible nitric oxide synthase. Bone Joint Res 2017:6:90-97.
Riggs, BL, Khosla, S, Melton, LJ. Sex Steroids and the Construction and Conservation of the Adult Skeleton. Endocrine Reviews 2002;23(3):279-302
Sandner P, Berger P, Zenzmaier C. The Potential of sGC Modulators for the Treatment of Age-Related Fibrosis: A Mini-Review. Gerontology. 2017;63(3):216-227
Schmidt HH, Hofmann F, Stasch JP. Handbook of Experimental Pharmacology 191. cGMP: generators, effectors and therapeutic implications. Preface. Handb Exp Pharmacol. 2009;(191)
Stasch JP et al., NO-independent regulatory site on soluble guanylate cyclase. Nature 2001, 410:212-215
Stasch JP and Hobbs AJ. NO-independent, haem-dependent soluble guanylate cyclase stimulators. Handb. Exp. Pharmacol. 2009, 191, 277 - 308
Stasch JP et al. NO- and haem-independent activation of soluble guanylyl cyclase: molecular basis and cardiovascular implications of a new pharmacological principle. Br. J. Pharmacol. 2002, 136(5):773-783
Stasch JP et al. Targeting the heme-oxidized nitric oxide receptor for selective vasodilatation of diseased blood vessels. J. Clin. Invest. 2006, 116(9):2552-61
Turner CH, Burr DB (1993). Basic biomechanical measurements of bone: a tutorial. Bone 14(4):595-608
van Dijk FS, Cobben JM, Kariminejad A, Maugeri A, Nikkels PG, van Rijn RR, Pals G. Osteogenesis Imperfecta: A Review with Clinical Examples. Mol Syndromol. 2011 Dec;2(1):1-20

## Claims

1. At least one sGC stimulator or sGC activator for use in the treatment and/or prophylaxis of bone diseases selected from the group consisting of osteogenesis imperfecta (OI), bone fractures, impaired bone healing, rickets, osteomalacia, avascular bone necrosis, and Paget disease.

2. At least one sGC stimulator or sGC activator according to claim 1 for use in the treatment and/or prophylaxis of osteogenesis imperfecta (OI).

3. At least one sGC stimulator or sGC activator according to claim 1 for use in the treatment and/or prophylaxis of bone diseases selected from the group consisting of bone fractures and impaired bone healing.

4. At least one sGC stimulator or sGC activator according to claim 1 for use in the treatment and/or prophylaxis of bone diseases selected from the group consisting of rickets, osteomalacia, avascular bone necrosis, and Paget disease.

5. At least one sGC stimulator or sGC activator for use in the treatment and/or prophylaxis of bone diseases selected from the group consisting of osteoporosis, primary osteoporosis, and secondary osteoporosis.

6. At least one sGC stimulator or sGC activator for use according to claim 5, wherein the osteoporosis, primary osteoporosis, and secondary osteoporosis is not caused by a deficiency of sex hormones.

7. At least one sGC stimulator for use according to any of claims 1 to 6, wherein the at least one sGC stimulator is selected from the group consisting of
2-[1-(2-fluorobenzyl)-1H-pyrazolo[3,4-b]pyridin-3-yl]-5-(4-morpholinyl)-4,6-pyrimidinediamine
2-[1-(2-fluorobenzyl)-1H-pyrazolo[3,4-b]pyridin-3-yl]-5-(4-pyridinyl)-4-pyrimidineamine
methyl 4,6-diamino-2-[1-(2-fluorobenzyl)-1H-pyrazolo[3,4-b]pyridin-3-yl]-5-pyrimidinyl(methyl)carbamate (Riociguat)
methyl 4,6-diamino-2-[1-(2-fluorobenzyl)-1H-pyrazolo[3,4-b]pyridin-3-yl]-5-pyrimidinylcarbamate (Nelociguat)
methyl {4,6-diamino-2-[5-fluoro-1-(2-fluorobenzyl)-1H-pyrazolo[3,4-b]pyridine-3-yl]pyrimidin-5-yl}carbamate (Vericiguat)
methyl {4,6-diamino-2-[5-fluoro-1-(2-fluorobenzyl)-1H-pyrazolo[3,4-b]pyridine-3-yl]pyrimidin-5-yl}methylcarbamate
3-(4-amino-5-cyclopropylpyrimidin-2-yl)-1-(2-fluorobenzyl)-1*H*-pyrazolo[3,4-*b*]pyridine (BAY 41-2272)
2-{5-fluoro-1-[(3-fluoropyridin-2-yl)methyl]-1H-pyrazolo[3,4-b]pyridin-3-yl}-5-methyl-5-(trifluoromethyl)-4-[(3,3,3-trifluoropropyl)amino]-5,7-dihydro-6H-pyrrolo[2,3-d]pyrimidin-6-one and
1,1,1,3,3,3-Hexafluoro-2-[({5-fluoro-2-[1-(2-fluorobenzyl)-5-(1,2-oxazol-3-yl)-1H-pyrazol-3-yl]-4-pyrimidinyl}amino)methyl]-2-propanol (Praliciguat)

8. At least one sGC stimulator for use according to claim 7, wherein the at least one sGC stimulator is selected from the group consisting of
methyl 4,6-diamino-2-[1-(2-fluorobenzyl)-1H-pyrazolo[3,4-b]pyridin-3-yl]-5-pyrimidinyl(methyl)carbamate (Riociguat)
methyl {4,6-diamino-2-[5-fluoro-1-(2-fluorobenzyl)-1H-pyrazolo[3,4-b]pyridine-3-yl]pyrimidin-5-yl}carbamate (Vericiguat)
3-(4-amino-5-cyclopropylpyrimidin-2-yl)-1-(2-fluorobenzyl)-1*H*-pyrazolo[3,4-*b*]pyridine (BAY 41-2272) and
1,1,1,3,3,3-Hexafluoro-2-[({5-fluoro-2-[1-(2-fluorobenzyl)-5-(1,2-oxazol-3-yl)-1H-pyrazol-3-yl]-4-pyrimidinyl} amino)methyl] -2-propanol (Praliciguat)

9. At least one sGC activator for use according to any of claims 1 to 5, wherein the at least one sGC activator is selected from the group consisting of
3-(4-chloro-3-{[(2S,3R)-2-(4-chlorophenyl)-4,4,4-trifluoro-3-methylbutanoyl]amino}phenyl)-3-cyclopropylpropanoic acid
5-{[2-(4-carboxyphenyl)ethyl][2-(2-{[3-chloro-4'-(trifluoromethyl)biphenyl-4-yl]methoxy}phenyl)ethyl]amino}-5,6,7,8-tetrahydroquinoline-2-carboxylic acid and
5-{(4-carboxybutyl)[2-(2-{[3-chloro-4'-(trifluoromethyl)biphenyl-4-yl]methoxy}phenyl)ethyl]amino}-5,6,7,8-tetrahydroquinoline-2-carboxylic acid

10. At least one sGC activator for use according to claim 9, wherein the sGC activator is
3-(4-chloro-3-{[(2S,3R)-2-(4-chlorophenyl)-4,4,4-trifluoro-3-methylbutanoyl]amino}phenyl)-3-cyclopropylpropanoic acid

11. Combination of at least one sGC stimulator or sGC activator and at least one compound selected from the group consisting of inhibitors of phosphodiesterases (PDE) 1, 2 and/or 5, calcium, vitamin D and metabolites of vitamin D, bisphosphonates, selected from etidronate, clodronate, tiludronate, teriparatide, pamidronate, neridronate, olpadronate, alendronate, ibandronate, risedronate, and zoledronate, strontium ranelate, active ingredients suitable for hormone replacement therapy in osteoporosis, selected from estrogen and a combination of estrogen and progesterone, selective estrogen receptor modulators (SERMs), parathyroid hormone and analogs of parathyroid hormone, modulators of receptor activator of nuclear factor kappa-B ligand (RANKL), sclerostin inhibitors, and TGF-β inhibitors for use according to any of claims 1 to 10.

12. Combination of at least one sGC stimulator or sGC activator and at least one inhibitor of phosphodiesterase (PDE) 5, selected from the group consisting of sildenafil, vardenafil, tadalafil and avanafil for use according to any of claims 1 to 11.

13. Pharmaceutical composition comprising at least one sGC stimulator or sGC activator according to any of claims 1 to 10 in combination with one or more inert non-toxic pharmaceutically suitable excipients for use according to any of claims 1 to 10.

14. Pharmaceutical composition comprising a combination according to claims 11 or 12 in combination with one or more inert non-toxic pharmaceutically suitable excipients for use according to any of claims 1 to 10.
